(19) Europäisches Patentamt
European Patent Office
Office européen des brevets

(11) **EP 3 770 852 A1**

(12) **EUROPEAN PATENT APPLICATION**

(43) Date of publication:
**27.01.2021 Bulletin 2021/04**

(51) Int Cl.:
**G06T 7/00** *(2017.01)*  **G06T 7/62** *(2017.01)*

(21) Application number: **20187432.8**

(22) Date of filing: **23.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.07.2019 US 201962877786 P**

(71) Applicant: **iSchemaView, Inc.**
**Menlo Park, CA 94025 (US)**

(72) Inventors:
• **WIENECKE, Herman Allan**
**Scottsdale, AZ Arizona 85251 (US)**
• **BABIKER, Mohamed Haithem**
**Phoenix, AZ Arizona 85042 (US)**

(74) Representative: **Forresters IP LLP**
**Skygarden**
**Erika-Mann-Straße 11**
**80636 München (DE)**

Remarks:
Claims filed after the date of filing of the application (Rule 68(4) EPC).

(54) **SYSTEMS AND METHODS FOR SIMULATING BRAIDED STENT DEPLOYMENTS**

(57) Disclosed are methods and systems which can include a mathematical algorithm with software implementation and web deployment for use by a physician to plan treatment for deploying a braided stent in the neurovasculature of a patient. As the stent is deployed in a blood vessel, the crucial position of its proximal end is nonlinearly dependent on vessel diameter which typically varies in an irregular way along the a priori unknown length of the deployment. The systems and methods compute deployment path and deployed diameter along the path, allowing physicians in real-time to experiment with and visualize deployments for any combination of stent size and distal position.

EP 3 770 852 A1

Figure 15

## Description

FIELD OF THE DISCLOSURE

The present invention is generally related to endovascular medical device deployment simulations. More specifically, the present invention relates to systems and methods for selecting a correct flow diverter (FD), such as a stent, for a specific patient by predicting deployment of the FD in the blood vessel of the patient in real-time.

BACKGROUND

Flow diverters (FDs) are being used with increasing frequency for the treatment of cerebral aneurysms. The immediate goal of FD treatment is to promote hemodynamic stasis and thrombus formation within the aneurysmal sac via flow diversion. Several studies have shown impressively effective use of FD devices in treating small to large aneurysms (See Briganti F, Delehaye L, Leone G, et al. Flow diverter device for the treatment of small middle cerebral artery aneurysms. J Neurointerv Surg. 2016;8(3):287-294. doi:10.1136/neurintsurg-2014-011460; Bhogal P, Perez MA, Ganslandt O, Bäzner H, Henkes H, Fischer S. Treatment of posterior circulation non-saccular aneurysms with flow diverters: a single-center experience and review of 56 patients. J Neurointerv Surg. 2017;9(5):471-481. doi:10.1136/neurintsurg-2016-012781; and Saatci I, Yavuz K, Ozer C, Geyik S, Cekirge HS. Treatment of intracranial aneurysms using the pipeline flow-diverter embolization device: a single-center experience with long-term follow-up results. American Journal of Neuroradiology. 2012;33(8):1436-1446. doi:10.3174/ajnr.A3246).

Recently, the FDA also approved the expanded indication of FD products for a much wider range of aneurysm sizes and locations, paving the way for additional FD entries into the market (See U.S. Food and Drug Administration. P100018/S015 Pipeline™ Flex Embolization Device Approval Letter. https://www.accessdata.fda.gov/cdrh_docs/pdf10/P100018S015A.pdf. Published January 25, 2019).

Nevertheless, selection of the correct FD size remains challenging and is an important consideration in the context of treatment success. Oversizing FDs can lead to in-stent stenosis or poor device expansion (See Caroff J, Tamura T, King RM, et al. Phosphorylcholine surface modified flow diverter associated with reduced intimal hyperplasia. J Neurointerv Surg. 2018;10(11):1097-1101. doi:10.1136/neurintsurg-2018-013776; and Laurent G, Makoyeva A, Darsaut TE, et al. In vitro reproduction of device deformation leading to thrombotic complications and failure of flow diversion. Interv Neuroradiol. 19(4):432-437). Under-sizing can lead to device migration, prolapse into the aneurysm, and/or poor vessel coverage (See Tsai Y-H, Wong H-F, Hsu S-W. Endovascular management of spontaneous delayed migration of the flow-diverter stent. J Neuroradiol. December 2018. doi:10.1016/j.neurad.2018.11.004; and Al-Mufti F, Amuluru K, Cohen ER, et al. Rescue therapy for procedural complications associated with deployment of flow-diverting devices in cerebral aneurysms. Oper Neurosurg. 2018;15(6):624-633. doi:10.1093/ons/opy020).

The conventional approach to sizing FDs begins with measuring vessel diameters in images at the desired proximal and distal landing points. Specifically, lines projected onto 2D images are used to quantify the diameters. Next, the vessel length between the two points is estimated. These collective measurements are used to select a FD size that will hopefully appose well to the vessel wall and cover the deployment region. However, this approach to sizing can be challenging as vessel diameters may vary considerably along the trajectory of a vessel. FDs can also elongate by more than 50% of the nominal length indicated by labeling (See Narata AP, Blasco J, Roman LS, et al. Early results in flow diverter sizing by computational simulation: quantification of size change and simulation error assessment. Oper Neurosurg. 2018;15(5):557-566. doi: 10.1093/ons/opx288).

Thus, there remains a need to improve selection systems and methods for identifying the correct FD for a specific patient.

SUMMARY

The devices of the present invention have several features, no single one of which is solely responsible for its desirable attributes. Without limiting the scope of this invention as expressed by the claims which follow, its more prominent features will now be discussed briefly. After considering this discussion, and particularly after reading the section entitled "Detailed Description," one will understand how the features of this invention provide several advantages over current designs.

An aspect of the present disclosure provides a method for real-time sizing of flow diverters for cerebral aneurysm treatment. The method includes providing an image segmentation of a surface of a blood vessel network and initializing a centerline x(s) of a vessel in the blood vessel network and a maximum inscribed spherical diameter D(s) of the vessel along the centerline x(s). The method further includes determining a modified x(s) by smoothing x(s) and D(s) based at least in part on a cubic spline, identifying one or more bulge segments along x(s), and replacing each of the one or more bulge segments with a direct path segment. The method further includes determining a modified D(s) based on one or more of a rate and a limit.

In certain further aspects, the method includes determining a length for the flow diverter based at least in part on the modified x(s).

In certain further aspects, the method includes determining a length for the flow diverter based at least in part on the equation

$$L_0 = \int_0^S \frac{\sin(\beta_0)}{\sqrt{1 - (D(s)cos(\beta_0)/D_0)^2}} ds$$

.

In certain further aspects, the method includes determining a change in the length based at least in part on a push force.

In certain further aspects, the method includes determining a ratio of a cross-sectional area of the flow diverter to a cross-sectional area of the vessel at each point along the modified x(s).

In certain further aspects, the method includes wherein the direct path segment is a more direct smooth path which is still unaffected by the vessel.

In certain further aspects, the method includes wherein the direct path segment is a more likely path that will be followed by the flow diverter.

In certain further aspects, the method includes determining a pore density of a surface of the flow diverter.

An aspect of the present disclosure provides a system for real-time sizing of flow diverters for cerebral aneurysm treatment. The system comprises one or more processors configured to: provide an image segmentation of a surface of a blood vessel network, initialize a centerline x(s) of a vessel in the blood vessel network and a maximum inscribed spherical diameter D(s) of the vessel along the centerline x(s); determine a modified x(s) by, smoothing x(s) and D(s) based at least in part on a cubic spline, identifying one or more bulge segments along x(s), and replacing each of the one or more bulge segments with a direct path segment; and determine a modified D(s) based on one or more of a rate and a limit.

In certain further aspects, the system includes wherein the one or more processors is further configured to determine a length for the flow diverter based at least in part on the modified x(s).

In certain further aspects, the system includes wherein the one or more processors is further configured to determine a length for the flow diverter based at least

$$L_0 = \int_0^S \frac{\sin(\beta_0)}{\sqrt{1 - (D(s)cos(\beta_0)/D_0)^2}} ds \quad \text{in}$$

part on the equation

In certain further aspects, the system includes wherein the one or more processors is further configured to determine a change in the length based at least in part on a push force.

In certain further aspects, the system includes wherein the one or more processors is further configured to determine a ratio of a cross-sectional area of the flow diverter to a cross-sectional area of the vessel at each point along the modified x(s).

In certain further aspects, the system includes wherein the direct path segment is a more direct smooth path which is still unaffected by the vessel.

In certain further aspects, the system includes wherein the direct path segment is a more likely path that will be followed by the flow diverter.

In certain further aspects, the system includes wherein the one or more processors is further configured to determine a pore density of a surface of the flow diverter.

An aspect of the present disclosure provides a non-transitory computer readable storage medium having stored thereon instructions that, when executed, cause a computing device to: receive an image segmentation of a surface of a blood vessel network, initialize a centerline x(s) of a vessel in the blood vessel network and a maximum inscribed spherical diameter D(s) of the vessel along the centerline x(s); determine a modified x(s) by, smoothing x(s) and D(s) based at least in part on a cubic spline, identifying one or more bulge segments along x(s), and replacing each of the one or more bulge segments with a direct path segment; and determine a modified D(s) based on one or more of a rate and a limit.

In certain further aspects, the instructions, when executed, cause the at least one computing device to determine a length for the flow diverter based at least in part on the modified x(s).

In certain further aspects, the instructions, when executed, cause the at least one computing device to determine

$$L_0 = \int_0^S \frac{\sin(\beta_0)}{\sqrt{1 - (D(s)\cos(\beta_0)/D_0)^2}} ds$$

a length for the flow diverter based at least in part on the equation

In certain further aspects, the instructions, when executed, cause the at least one computing device to determine a change in the length based at least in part on a push force.

An aspect of the present disclosure provides a method for real-time sizing of flow diverters for cerebral aneurysm treatment. The method comprises providing an image segmentation of a surface of a blood vessel network; initializing a centerline x(s) of a vessel in the blood vessel network and a maximum inscribed predefined geometric shape D(s) of the vessel along the centerline x(s); determining a modified x(s) by, smoothing x(s) and D(s), identifying one or more bulge segments along x(s), and replacing each of the one or more bulge segments with a direct path segment; and determining a modified D(s).

An aspect of the present disclosure provides a system for real-time sizing of flow diverters for cerebral aneurysm treatment. The system comprises one or more processors configured to: provide an image segmentation of a surface of a blood vessel network, initializing a centerline x(s) of a vessel in the blood vessel network and a maximum inscribed predefined geometric shape D(s) of the vessel along the centerline x(s); determining a modified x(s) by, smoothing x(s) and D(s), identifying one or more bulge segments along x(s), and replacing each of the one or more bulge segments with a direct path segment; and determining a modified D(s).

Further aspects features and advantages of the present invention will become apparent from the detailed description that follows.

BRIEF DESCRIPTION OF THE DRAWINGS

These and other features, aspects, and advantages of the present invention will now be described in connection with embodiments of the present invention, in reference to the accompanying drawings. The illustrated embodiments, however, are merely examples and are not intended to limit the invention. Some embodiments will be described in conjunction with the appended drawings, where like designations denote like elements.

Embodiments of the invention will now be described, by way of example only, with reference to the accompanying drawings in which corresponding reference symbols indicate corresponding parts.

Figure 1 depicts a side view of an unconstrained braided stent of external diameter $D_{e0}$, mean diameter $D_0$, length $L_0$, and pitch angle $\beta_0$ in accordance with an exemplary embodiment of the present invention.

Figure 2 is a partial cross-section through the unconstrained braided stent of Figure 1 illustrating an external diameter $D_{e0}$, mean diameter $D_0$, and wires of diameter d.

Figure 3 is a representation of a portion of vasculature of a patient and shows vessel centerlines x(s) (blue, light blue, green, yellow, orange, and red) of the vasculature determined based on maximum inscribed spherical diameter (MISD).

Figure 4 is a representation of another portion of the vasculature and shows a series of spherical vessel diameters D(s) fit within the vasculature.

Figure 5 shows points from a portion of the original MISD from Figure 4 along with a smoothed MISD of the same portion.

Figure 6 is a graph of MISD v. arc length showing the smoothed MISD 40 and the original MISD.

Figure 7 illustrates bulge segments along the vessel centerline x(s) where the spherical vessel diameter D(s) of the MISD is greater than a maximum possible diameter of a deployed stent.

Figure 8 illustrates a predicted stent path or new path x(s) overlaid with the vessel centerline x(s) through the blood vessels of the patient showing that the predicted stent path deviates from the vessel centerline x(s) at one large bulge-segment location and two small bulge-segments locations along the vessel centerline x(s).

Figure 9 is a graph of MISD v. arc length similar to Figure 6 but with modified values for D(s) based on rate and limits.

Figure 10 illustrates a web portal page for treatment planning. The web portal page allows, in certain embodiments, physicians to visualize stent deployments while experimenting in real-time with different combinations of stent type, labeled diameter, labeled length, and/or distal landing point.

Figure 11 illustrates the web portal from Figure 10 and shows how UI/UX elements can be used to wary the amount of "push force" to apply to the stent.

Figure 12 illustrates the web portal from Figure 10 and shows how UI/UX elements can be used for visualizing apposition.

Figure 13 depicts a computer system for selecting a correct FD for a specific patient by predicting deployment of the FD in the blood vessel of the patient in real-time in accordance with an exemplary embodiment of the present invention.

Figure 14 is an exemplary representation of any of the modules of the computer system from Figure 13.

Figure 15 illustrates an exemplary method performed by the system for selecting a correct FD for a specific patient by predicting deployment of the FD in the blood vessel of the patient in real-time.

The various features illustrated in the drawings may not be drawn to scale. Accordingly, the dimensions of the various features may be arbitrarily expanded or reduced for clarity. In addition, some of the drawings may not depict all of the components of a given system, method or device. Finally, like reference numerals may be used to denote like features throughout the specification and figures.

DETAILED DESCRIPTION

In the following description, and for the purposes of explanation, numerous specific details are set forth in order to provide a thorough understanding of the various aspects of the invention. It will be understood, however, by those skilled in the relevant arts, that the present invention may be practiced without these specific details. In other instances, known structures and devices are shown or discussed more generally in order to avoid obscuring the invention. In many cases, a description of the operation is sufficient to enable one to implement the various forms of the invention, particularly when the operation is to be implemented in software. It should be noted that there are many different and alternative configurations, devices and technologies to which the disclosed inventions may be applied. The full scope of the inventions is not limited to the examples that are described below.

Analytic Relationship Between Stent Diameter and Length

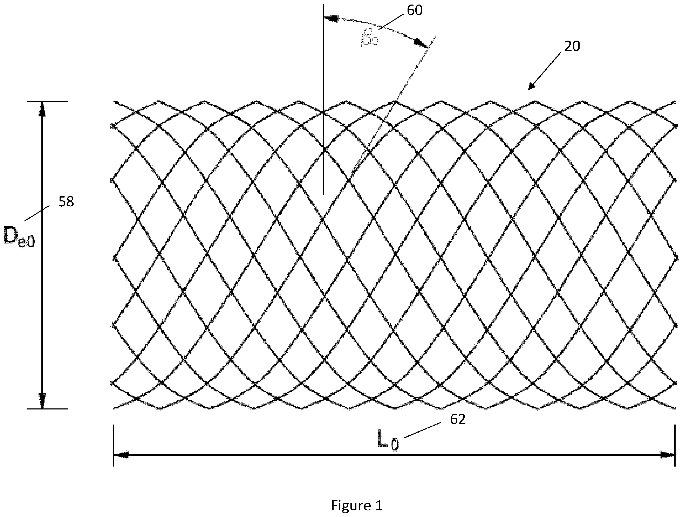

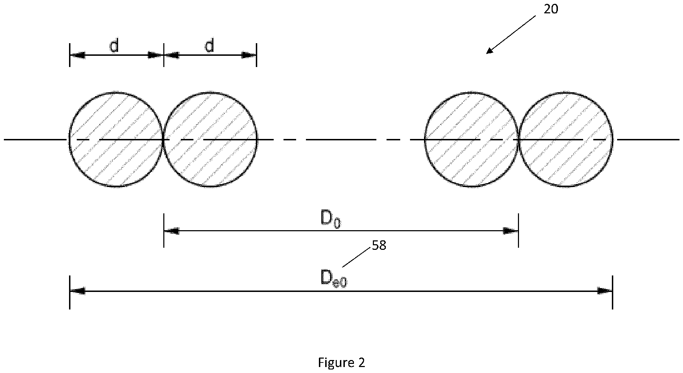

**Figure 1** depicts an unconstrained braided stent 20 of external diameter $D_{e0}$ 58, mean diameter $D_0$, length $L_0$ 62, and pitch angle $\beta_0$ 60 in accordance with an exemplary embodiment of the present invention. **Figure 2** is a partial cross-section through the unconstrained braided stent 20 of **Figure 1** illustrating an external diameter $D_{e0}$ 58, mean diameter $D_0$, and wires of diameter d.

Certain embodiments of the systems and methods disclosed herein for simulating braided stent deployment in neurovasculature are based on kinematics of braided stents. Kinematics can be derived from *Jedwab* (See Jedwab MR, Clerc CO. A study of the geometrical and mechanical properties of a self-expanding metallic stent-theory and experiment. Journal of Applied Biomaterials. 1993;4(1):77-85. doi:10.1002/jab.770040111). In certain embodiments, the kinematics rely on mathematical relationships between changes in diameter and changes in length of braided stents. For example, in certain embodiments, the relationship is initially derived from equations (1) and (2), below, from *Jedwab*:

$$(1) \quad \frac{D}{D_0} = \frac{\cos(\beta)}{\cos(\beta_0)}$$

$$(2) \quad \frac{L}{L_0} = \frac{\sin(\beta)}{\sin(\beta_0)}$$

where $D_0$, $L_0$, and $\beta_0$ are mean diameter, length, and pitch angle of an unconstrained cylindrical braided stent 20, and where D, L, and $\beta$ are those variables after a uniform change in the mean diameter.

Implementation

Equations (1) and (2) are derived for changes which are uniform along the stent length. Unlike equations (1) and (2), the systems and methods disclosed herein iimplement a differential form of the equations and integrates them as a function of arc lengths along the centerline x(s) 24 of a stent 20 with diameter D(s) 26.

$$L_0 = \int_0^s \frac{\sin(\beta_0)}{\sqrt{1 - (D(s)\cos(\beta_0)/D_0)^2}} ds$$

(3)

In this way, deployed length S is uniquely determined by equation (3). Prediction of S requires estimates for x(s) 24 of the stent 20 and D(s) 26 of the stent 20. In certain embodiments, estimates of x(s) 24 and D(s) 26 are determined by the following:

Initializing x(s) and D(s)

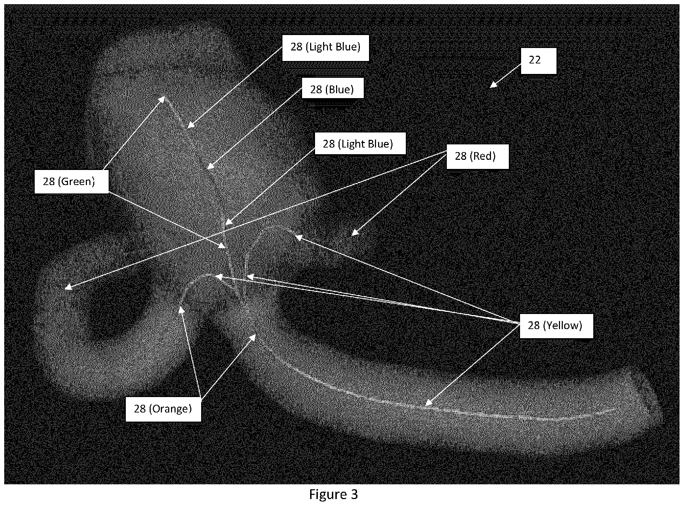

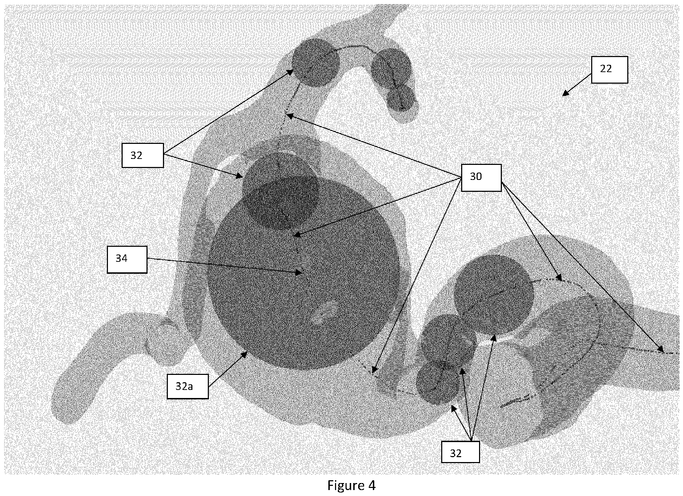

**Figure 3** is a representation of a portion of a vasculature 22 of a patient and shows vessel centerlines x(s) 28 (blue, light blue, green, yellow, orange, and red). In certain embodiments, the vessel centerlines x(s) 28 are determined at least in part based on one or more predefined geometric shapes. In certain embodiments, at least one of the predefined geometric shapes is a sphere. In certain embodiments, at least one of the predefined geometric shapes is an ellipse. Of course the method is not limited to constructing and fitting spheres or ellipses and may include other shapes as well as combinations of different shapes. In the embodiment illustrated in **Figure 4,** the predefined geometric shape within the vasculature 22 is a maximum inscribed spherical diameter (MISD) 30. In certain embodiments, the MISD 30 defines the vessel centerlines x(s) 28 and includes a plurality of spherical vessel diameters D(s) 32. The colors representing the vessel centerlines x(s) 28 in **Figure 3** are exemplary and intended to show values of the spherical vessel diameter D(s) 32 vary along the vessel centerlines x(s) 28 (see **Figure 4).** For example, the change in color from light blue to blue and then back to light blue again indicates the value of the spherical vessel diameter D(s) 32 increases (blue) and then decreases (light blue) at points along the vessel centerlines x(s) 28.

**Figure 4** is a representation of another portion of the vasculature 22 and shows a series of spherical vessel diameters D(s) 32 fit within the vasculature 22. While eight spherical vessel diameters D(s) 32 are illustrated in **Figure 4,** the methods and systems disclosure herein are not limited to the illustrated number. The series of spherical vessel diameters D(s) 32 can include more or less than what is illustrated in **Figure 4.**

In certain embodiments where the predefined geometric shape is a sphere, the series of spherical vessel diameters D(s) 32 together define the MISD 30 along the vasculature 22. The MISD 30 shown in **Figure 4** determines the vessel centerlines x(s) 28 shown in **Figure 3.** The spherical vessel diameters D(s) 32 of the MISD 30 can change at points along the vessel centerlines x(s) 28. In certain embodiments, a center 34 of the spherical vessel diameter D(s) 32a is employed to at least in part define the MISD 30 and can be associated with a point along the vessel centerlines x(s) 28 in **Figure 3.** A larger MISD 30 at a given point along the vessel centerline x(s) 28 indicates the relative value of the spherical vessel diameter D(s) 32 is also greater at that point.

As will be explained below, in certain embodiments, the centerline x(s) 24 of the stent 20 is initialized as the vessel centerline x(s) 28, and the diameter D(s) 26 of the stent 20 is initialized as the MISD 30 of the vessel along the vessel centerline x(s) 28.

In certain embodiments, the vessel centerline x(s) 28 and the MISD 30 are irregularly-sampled piecewise-linear functions computed by systems and methods disclosed in filed U.S. Patent Applications SYSTEMS AND METHODS FOR ANALYTICAL DETECTION OF ANEURYSMS, Application No.: 16/516136, filed on July 18, 2019, SYSTEMS AND METHODS FOR MEASUREMENT ANALYSIS OF ANEURYSMS, Application No.: 16/516150, filed July 18, 2019; and SYSTEMS AND METHODS FOR ANALYTICAL COMPARISON AND MONITORING OF ANEURYSMS, Application No.: 16/516140, filed July 18, 2019 from the surface mesh derived from a segmented image of the vasculature. The entire disclosures of the listed pending U.S. Patent Applications above are hereby incorporated by reference in their entireties.

Smoothing x(s) and D(s)

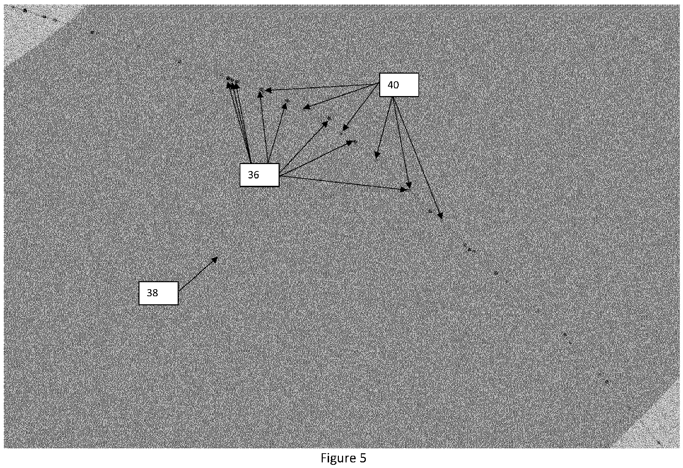

**Figure 5** shows points 36 from a portion 38 of the original MISD 30 from **Figure 4** along with a smoothed MISD 40 of the same portion 38. In certain embodiments, the smoothed MISD 40 is determined by slightly smoothing and sampling one or more of the points 36 from the original MISD 30. In certain embodiments, the original MISD 30 is sampled at a parameter value of 0.2mm to define the smoothed MISD 40. In other embodiments, the parameter value is smaller or larger than 0.2mm.

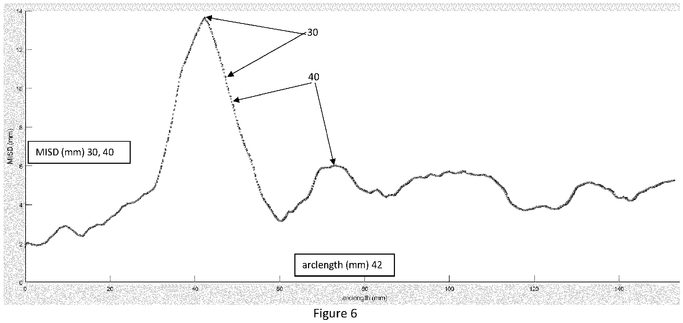

**Figure 6** is a graph of MISD 30, 40 v. arc length 42 showing the smoothed MISD 40 and the original MISD 30. In certain embodiments, the vessel centerline x(s) 28 and the original MISD 30 are each slightly smoothed (residual sum of squares - RSS < 0.01mm) with a cubic spline parameterized by the arc lengths along the vessel centerline x(s) 28. In certain embodiments, each is regularly sampled at small intervals of length Δs.

Identifying Bulge Segments

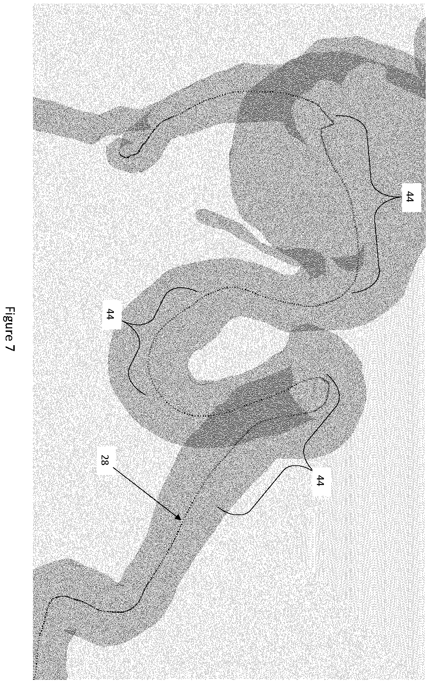

**Figure 7** illustrates bulge segments 44 along the vessel centerline x(s) 28 where the spherical vessel diameter D(s) 32 of the MISD 30, 40 is greater than a maximum possible diameter of a deployed stent 20. In certain embodiments, the method identifies segments 44 along the vessel centerline x(s) 28 where the segment 44 could deviate from a more direct, realistic path for the stent 20. In certain embodiments, this occurs at locations where the deployed path of the stent 20 is not determined by the vessel. For example, these locations can occur where the spherical vessel diameter D(s) 32 of the MISD 30, 40 is larger than a maximum attainable external diameter of the deployed stent 20. In such a case, the segment 44 is identified as a bulge segment 44. In certain embodiments, the bulge segment 44 is identified with a sequence of data points along the vessel centerline x(s) 28 where $D(s) > D0+2d-\epsilon$ for fixed $\epsilon > 0$ and where $\alpha D(s_j) > D_0+2d-\epsilon$ for fixed $\alpha < 1$ at a point $s_j$ of the sequence. The second condition is not necessary, but it is computationally efficient because it prevents analysis of segments where a size of the bulge segment 44 is small enough that the stent 20 cannot appreciably deviate from the vessel centerline x(s) 28.

Replacing the Centerlines of Bulge Segments with a Direct Path

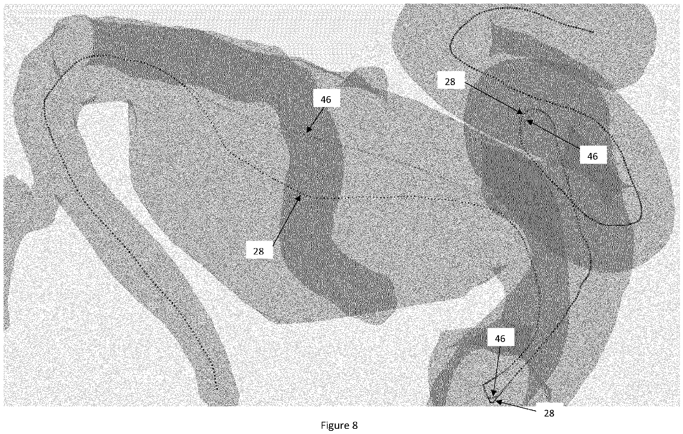

**Figure 8** illustrates a predicted stent path or new path x(s) 46 overlaid with the vessel centerline x(s) 28 through the blood vessels of the patient showing that the predicted stent path 46 deviates from the vessel centerline x(s) 28 at one large bulge-segment location and two small bulge-segments locations along the vessel centerline x(s) 28. In certain embodiments, unlikely paths along the vessel centerline x(s) 28 that pass through a bulge segment 44 are replaced with a predicted stent path or new path x(s) 46. The predicted stent path or new path x(s) 46 can be a more direct smooth path which is still unaffected by the vessel. In certain embodiments, the resulting predicted stent path or new path x(s) 46 is a more likely path that will be followed by the stent 20.

In certain embodiments, the predicted stent path or new path x(s) 46 is computed iteratively. For example, in certain embodiments, each iteration ignores the data along the bulge segments 44, fits the remaining data in x and D with cubic splines parameterized by arc lengths, samples each spline at intervals of length $\Delta s$, and checks the predicted stent path or new path x(s) 46 through each bulge segment 44 for any penetration of the vessel mesh by a stent 20 fully expanded around the predicted stent path or new path x(s) 46. If penetration exists then, in certain embodiments, each bulge segment 44 with penetration is shortened by ~4% at each end, x(s) and D(s) are restored to their original values, and a new iteration begins. Of course, the stent 20 can be shortened by amounts other than 4% and still fall within the scope of this disclosure.

When mesh penetration by the fully expanded stent 20 is no longer detected, the predicted stent path or new path x(s) 46 represents a more direct and realistic stent path, and D(s) represents vessel MISD 30, 40 except along bulge segments 44 where it is an approximation used as a quick test for penetration. In certain embodiments, only if the test is negative, is a more expensive test used.

Modifying D(s) Rate and Limits

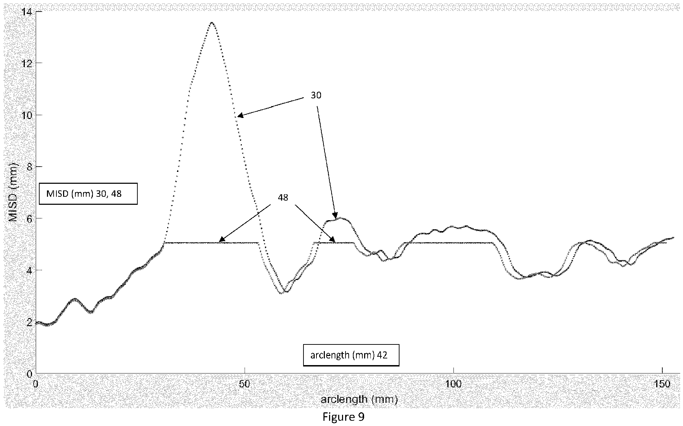

**Figure 9** is a graph of MISD 30, 48 v. arc length similar to **Figure 6** but with modified values for D(s) based on rate and limits. In certain embodiments, modified D(s) of MISD 48 represents the mean diameter of the deployed stent 20. For example, D(s) can be modified to (A) represent mean instead of external diameter by subtracting 2d; (B) not exceed the maximum possible diameter by applying an upper threshold of D0-$\epsilon$; and/or (C) not vary more rapidly than possible by applying equal lower and upper thresholds to D(s)'s derivative. For example, the upper threshold can be applied pointwise, forward through D(s). For example, the lower threshold can be applied pointwise, backward through D(s).

Computing the 4D Array of Deployment Lengths

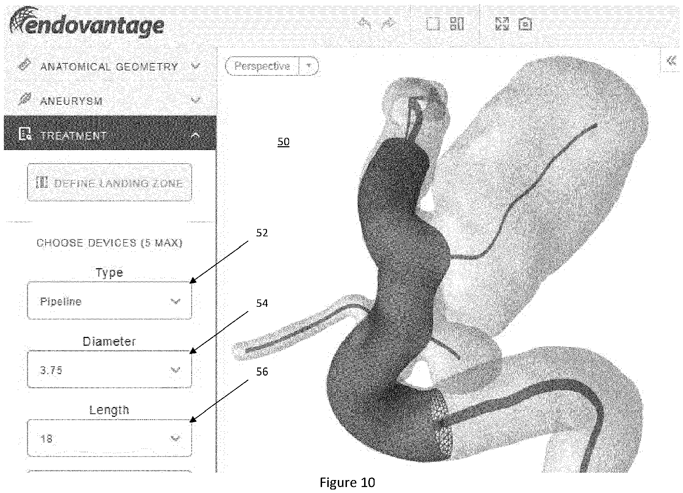

**Figure 10** illustrates a web portal page 50 for treatment planning. The web portal page 50 allows, in certain embodiments, physicians to visualize stent deployments while experimenting in real-time with different combinations of stent type 52, labeled diameter 54, labeled length 56, and/or distal landing point. In certain embodiments, the labeled diameter 54 and the length 56 refer to dimensions of the stent 20 as constrained by manufacturer packaging. Unconstrained by the packaging, the stent 20 slightly increases by a predictable amount from the labeled diameter 54 to its unconstrained diameter $D_0$ 58. A labeled pitch angle is the unconstrained pitch angle $\beta_0$ 60, which along with the two diameters 54, 58 and labeled length 56 is used in equations (1) and (2) to find unconstrained length $L_0$ 62.

Once the stent path and diameter are computed as described above, equation (3) is used to compute deployed stent length S for all possible stent distal points along the discretized path x(sj). In certain embodiments, this same process is repeated for all vessels in the vasculature 22 of the patient and can also be repeated for all commercially labelled stent sizes, yielding the following data structures: xijk is kth point of the centerline for the stent of jth labelled diameter on ith vessel; Dijk is mean stent diameter at xijk; Sijkm is deployed length for a stent of jth labelled diameter and mth labeled length deployed at point xijk of ith vessel. In certain embodiments, the data is available to the web page portal 50. In certain embodiments, a physician can experiment in real-time with and visualize different stent 20 deployments in the vasculature 22 of the patient, as shown in **Figure 10.** In certain embodiments where many possible deployment scenarios have been precomputed, the web page portal 50 indexes the results and provides responses to the physician in real-time.

Computing Stent Length at Different Levels of Push Force

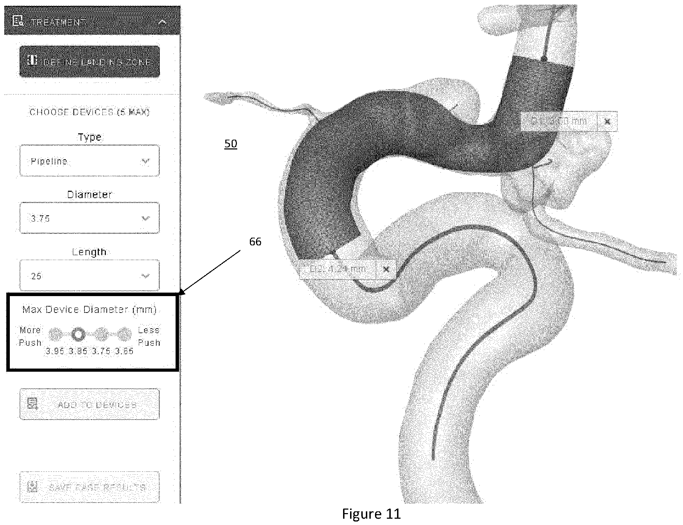

**Figure 11** illustrates the web portal 50 from **Figure 10** and shows how user interface (UI) and user experience (UX) elements can be used to vary the simulated amount of "push force" 66 being applied to the stent 20. A deployed length of the stent 20 can be sensitive to the amount of force used to push the stent 20 forward during deployment. In certain embodiments, applying more force to the stent 20 leads to: (A) a compression of the wires of the stent 20; (B) an increase in the diameter of the stent 20; and/or (C) a reduction in the length of the stent 20. Less force has the opposite effect. In certain embodiments, applying less force to the stent 20 mitigates against: (A) compression of the wires of the stent 20; (B) increasing the diameter of the stent 20; and/or (C) reducing the length of the stent 20.

The "push force" 66 effects on stent length can be modeled by varying $\varepsilon$ in the upper threshold $D_0$-$\varepsilon$ imposed on stent diameter D(s) (see Modifying D(s) rate and limits). In certain embodiments, decrementing $\varepsilon$ from 0.0 to 0.3 mm produces multiple sets of the deployment arrays $x_{ijk}$, $D_{ijk}$, and $S_{ijkm}$. In certain embodiments, the deployment arrays represent all possible deployments for a particular diameter expansion limit. In certain embodiments, the diameter expansion limit is a quantitative surrogate for push force 66. The different sets of deployment arrays enable the physician to visualize effects of different levels of push force 66, as shown in **Figure 11.**

Computing Stent-to-Vessel Apposition

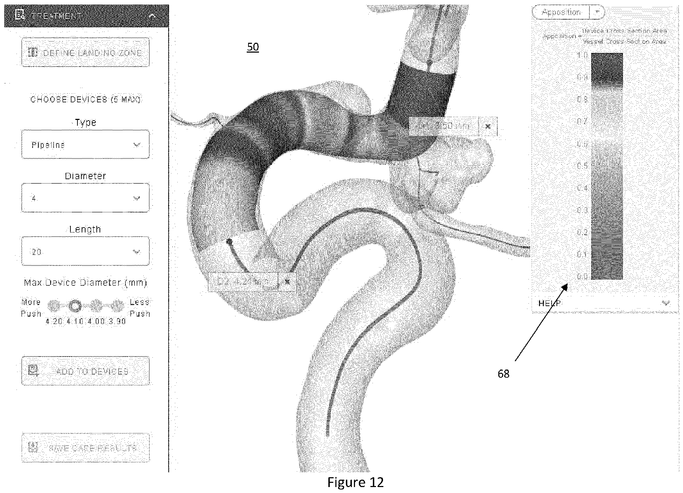

**Figure 12** illustrates the web portal 50 from **Figure 10** and shows how UI/UX elements can be used for visualizing apposition 68. In certain embodiments, apposition is defined as the ratio of device cross-sectional area to vessel cross-sectional area at each point along the discretized path $x(_{sj})$. Device cross-sectional area is calculated at point $x_{ijk}$ using equation (4) and vessel cross-sectional area is computed by calculating the polygonal area of a cross-section of the vessel mesh that is perpendicular to the discretized path x(sj) at point $x_{ijk}$.

$$\frac{\pi(D_{ijk})^2}{4} \quad (4)$$

In certain embodiments, apposition is calculated for all vessels in the patient vasculature and/or for all commercially labeled stent sizes. The calculation results in the data structure $A_{ijk}$ for a stent of $j^{th}$ labeled diameter deployed at point $x_{ijk}$ of $i^{th}$ vessel. As is shown in **Figure 12,** the apposition arrays (one for each push force level) allow physicians to visualize apposition 68 as a color-map.

Computing Pore Density

In certain embodiments, pore density is defined as pores/mm$^2$ on the surface of the stent 20. In certain embodiments, the pore density is calculated by applying equation (1) to determine pitch angle $\beta(s)$ for a straight stent of diameter D(s). In certain embodiments to account for bending, the pitch angle is computed around the stent 20 as $\sin(\beta(s,\theta)) = (1 + 0.5*D(s)*\cos(0)/p(s)) * \sin(\beta(s))$, where $\theta$ is angle around the tangent to x(s) measured from outward unit normal n(s), and where $\rho(s)$ is the radius of curvature along x(s). Since relative change in $\sin(\beta)$ is equivalent to relative change in length (equation (2)), this equation for $\sin(\beta(s,\theta))$ is analogous for mechanical strain in beam bending. D(s) and $\beta(s,\theta)$ determine area $a(s,\theta)$ of the stent's rhomboidal pores, and pore density $p(s,\theta) = 1/a(s,\theta)$. In certain embodiments, the pore density is computed for all vessels and stent sizes yielding the data structure $p_{ijkm}$ for pore density at the $m^{th}$ angle around a stent of $j^{th}$ labeled diameter deployed at point $x_{ijk}$ of $i^{th}$ vessel. The web portal 50 illustrated in **Figures 10-12** can be configured to allow the physician to visualize pore density as a color-map by displaying the pore density arrays (one for each push force level).

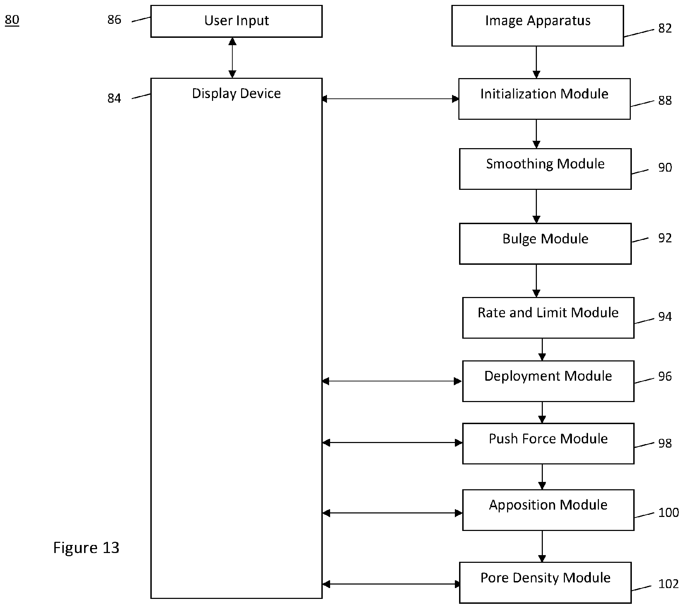

**Figure 13** depicts a computer system 80 for selecting a correct FD 20 for a specific patient by predicting deployment of the FD 20 in the blood vessel of the patient in real-time in accordance with an exemplary embodiment of the present invention. In certain embodiments, the computer system 80 comprises one or more of a initialization module 88, a smoothing module 90, a bulge module 92, a rate and limit module 94, a deployment module 96, a push force module 98, an apposition module 100, and a pore density module 102. In certain embodiments, the computer system 80 comprises one or more of an image apparatus 82, a display device 84, and a user input 86.

As explained above with respect to **Figure 3,** the initialization module 88 is configured to determine the vessel centerlines x(s) 28 at least in part based on a maximum inscribed spherical diameter (MISD) 30 within the vasculature 22. In certain embodiments, the MISD 30 defines the vessel centerlines x(s) 28 and includes a plurality of spherical vessel diameters D(s) 32. In certain embodiments, the series of spherical vessel diameters D(s) 32 together define the MISD 30 along the vasculature 22.

In certain embodiments, the centerline x(s) 24 of the stent 20 is initialized as the vessel centerline x(s) 28, and the diameter D(s) 26 of the stent 20 is initialized as the MISD 30 of the vessel along the vessel centerline x(s) 28.

As explained above with respect to **Figures 5** and **6,** the smoothing module 90 is configured to smooth the original MISD 30 from **Figure 4.** In certain embodiments, the smoothed MISD 40 is determined by slightly smoothing and sampling one or more of the points 36 from the original MISD 30. In certain embodiments, the original MISD 30 is sampled at a parameter value of 0.2mm to define the smoothed MISD 40. In other embodiments, the parameter value is smaller or larger than 0.2mm.

In certain embodiments, the smoothing module 90 slightly smooths the vessel centerline x(s) 28 and the original MISD 30 (residual sum of squares - RSS < 0.01mm) with a cubic spline parameterized by the arc lengths along the vessel centerline x(s) 28. In certain embodiments, each is regularly sampled at small intervals of length $\Delta$s.

As explained above with respect to **Figures 7** and **8,** the bulge module 92 is configured to identify segments 44 along the vessel centerline x(s) 28 where the segment 44 could deviate from a more direct, realistic path for the stent 20. In certain embodiments, this occurs at locations where the deployed path of the stent 20 is not determined by the vessel. For example, these locations can occur where the spherical vessel diameter D(s) 32 of the MISD 30, 40 is larger than a maximum attainable external diameter of the deployed stent 20. In such a case, the bulge module 92 identifies the segment as a bulge segment 44. In certain embodiments, the bulge module 92 identifies the bulge segment 44 with a sequence of data points along the vessel centerline x(s) 28 where D(s) > D0+2d-$\varepsilon$ for fixed $\varepsilon$ > 0 and where $\alpha$D(sj) > D0+2d-$\varepsilon$ for fixed $\alpha$ < 1 at a point sj of the sequence. The second condition is not necessary, but it is computationally efficient because it prevents analysis of segments where a size of the bulge segment 44 is small enough that the stent 20 cannot appreciably deviate from the vessel centerline x(s) 28.

In certain embodiments, the bulge module 92 is configured to replace the segment 44 with a direct path. In certain embodiments, the bulge module 92 predicts a stent path or new path x(s) 46 and overlays the new path with the vessel centerline x(s) 28 through the blood vessels of the patient. In certain embodiments, the bulge module 92 replaces unlikely paths along the vessel centerline x(s) 28 that pass through the bulge segment 44 with the predicted stent path or new path x(s) 46. The predicted stent path or new path x(s) 46 can be a more direct smooth path which is still unaffected by the vessel. In certain embodiments, the resulting predicted stent path or new path x(s) 46 is a more likely path that will be followed by the stent 20.

In certain embodiments, the bulge module 92 computes the predicted stent path or new path x(s) 46 iteratively. For example, in certain embodiments, each iteration ignores the data along the bulge segments 44, fits the remaining data in x and D with cubic splines parameterized by arc lengths, samples each spline at intervals of length $\Delta$s, and checks the predicted stent path or new path x(s) 46 through each bulge segment 44 for any penetration of the vessel mesh by a stent 20 fully expanded around the predicted stent path or new path x(s) 46. If penetration exists then, in certain embodiments, the bulge module 92 shortens each bulge segment 44 by ~4% at each end, x(s) and D(s) are restored to their original values, and a new iteration begins. Of course, the stent 20 can be shortened by amounts other than 4% and still fall within the scope of this disclosure.

When mesh penetration by the fully expanded stent 20 is no longer detected the bulge module 92, the predicted stent path or new path x(s) 46 represents a more direct and realistic stent path, and D(s) represents vessel MISD 30, 40 except along bulge segments 44 where it is an approximation used as a quick test for penetration. In certain embodiments, only if the test is negative, is a more expensive test used.

As explained in more detail above with respect to **Figure 9,** the rate and limit module 94 is configured to modify D(s) to (A) represent mean instead of external diameter by subtracting 2d; (B) not exceed the maximum possible diameter by applying an upper threshold of D0-$\varepsilon$; and/or (C) not vary more rapidly than possible by applying equal lower and upper thresholds to D(s)'s derivative. For example, the rate and limit module 94 can apply pointwise an upper threshold, forward through D(s). For example, the rate and limit module 94 can apply pointwise a lower threshold, backward through D(s).

As explained in more detail above with respect to **Figure 10,** the deployment module 96 is configured to allow physicians to visualize stent deployments while experimenting in real-time with different combinations of stent type 52, labeled diameter 54, labeled length 56, and/or distal landing point. In certain embodiments, the labeled diameter 54 and the length 56 refer to dimensions of the stent 20 as constrained by manufacturer packaging. Unconstrained by the packaging, the stent 20 slightly increases by a predictable amount from the labeled diameter 54 to its unconstrained diameter D0 58. A labeled pitch angle is the unconstrained pitch angle β0 60, which along with the two diameters 54, 58 and labeled length 56 is used in equations (1) and (2) to find unconstrained length L0 62.

Once the stent path and diameter are computed as described above, equation (3) is used to compute deployed stent length S for all possible stent distal points along the discretized path x(sj). In certain embodiments, the data is available to the web page portal 50. In certain embodiments, a physician can experiment in real-time with and visualize different stent 20 deployments in the vasculature 22 of the patient, as shown in **Figure 10.** In certain embodiments where many possible deployment scenarios have been precomputed, the web page portal 50 indexes the results and provides responses to the physician in real-time.

As explained in more detail above with respect to **Figure 11,** the push force module 98 is configured to allow physicians to vary the simulated amount of "push force" 66 being applied to the stent 20. A deployed length of the stent 20 can be sensitive to the amount of force used to push the stent 20 forward during deployment. In certain embodiments, applying more force to the stent 20 leads to: (A) a compression of the wires of the stent 20; (B) an increase in the diameter of the stent 20; and/or (C) a reduction in the length of the stent 20. Less force has the opposite effect. In certain embodiments, applying less force to the stent 20 mitigates against: (A) compression of the wires of the stent 20; (B) increasing the diameter of the stent 20; and/or (C) reducing the length of the stent 20.

The "push force" 66 effects on stent length can be modeled by varying ε in the upper threshold D0-ε imposed on stent diameter D(s) (see Modifying D(s) rate and limits). In certain embodiments, the push force module 98 decrements ε from 0.0 to 0.3 mm to produce multiple sets of the deployment arrays xijk, Dijk, and Sijkm. In certain embodiments, the deployment arrays represent all possible deployments for a particular diameter expansion limit. In certain embodiments, the diameter expansion limit is a quantitative surrogate for push force 66. The different sets of deployment arrays provided by the push force module 98 enable the physician to visualize effects of different levels of push force 66.

As explained in more detail above with respect to **Figure 12,** the apposition module 102 is configured to visualize apposition. In certain embodiments, the apposition module 102 can calculate a device cross-sectional area at point $x_{ijk}$ using equation (4) and can compute a vessel cross-sectional area by calculating the polygonal area of a cross-section of the vessel mesh that is perpendicular to the discretized path x(sj) at point $x_{ijk}$.

In certain embodiments, the apposition module 102 calculates apposition for all vessels in the patient vasculature and/or for all commercially labeled stent sizes. The calculation results in the data structure $A_{ijk}$ for a stent of $j^{th}$ labeled diameter deployed at point $x_{ijk}$ of $i^{th}$ vessel. The apposition arrays (one for each push force level) allow physicians to visualize apposition as a color-map 68.

As explained in more detail above, the pore density module 102 is configured to calculate pore density by applying equation (1) to determine pitch angle β(s) for a straight stent of diameter D(s). In certain embodiments to account for bending, the pore density module 102 computes the pitch angle around the stent 20 as sin(β(s,θ)) = (1 + 0.5*D(s)*cos(θ)/p(s)) * sin( β(s) ), where θ is angle around the tangent to x(s) measured from outward unit normal n(s), and where ρ(s) is the radius of curvature along x(s). Since relative change in sin(β) is equivalent to relative change in length (equation (2)), this equation for sin(β(s,θ)) is analogous for mechanical strain in beam bending. D(s) and β(s,θ) determine area a(s,θ) of the stent's rhomboidal pores, and pore density p(s,θ) = 1/a(s,θ). In certain embodiments, the pore density module 102 computes the pore density for all vessels and stent sizes yielding the data structure pijkm for pore density at the mth angle around a stent of jth labeled diameter deployed at point xijk of ith vessel.

Some embodiments of the system 80, which is used for selecting a correct FD 20 for a specific patient by predicting deployment of the FD 20 in the blood vessel of the patient in real-time, include some or all of the components shown in **Figure** 13. The image apparatus 82 is used to determine, analyze, and/or display vascular information for a patient. In certain embodiments, the image apparatus 82 is a Computed Tomography (CT) or Magnetic Resonance (MR) apparatus. In certain embodiments, the image apparatus 82 produces 3D image data or scans for the patient. In some examples, this data is in the form of a series of cross-sectional data scans. This data is represented, for instance through a process of resampling or other form of image processing. In certain embodiments, the image apparatus 82 provides image data or scans to the initialization module 88 for further processing.

In the system shown in **Figure 13,** the display device 84 is used to produce a presentation image for presentation to a healthcare provider. For instance, the presentation image shows the results of one or more of the initialization module 88, the deployment module 96, the push force module 98, the apposition module 100, and the pore density module 102.

In the system shown in **Figure 13,** the user input 86 allows the healthcare provider to review, analyze, modify, and select at least a portion of the results from one or more of the initialization module 88, the deployment module 96, the push force module 98, the apposition module 100, and the pore density module 102.

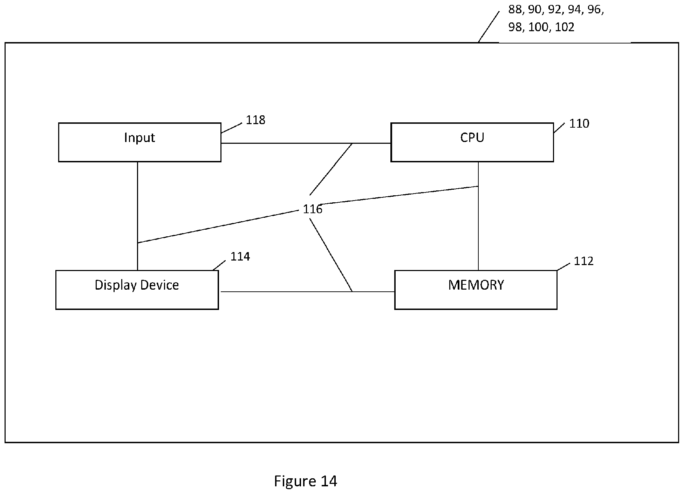

**Figure 14** is an exemplary representation of any of the modules of the computer system 80 including the one or more of the initialization module 88, the smoothing module 90, the bulge module 92, the rate and limit module 94, the deployment module 96, the push force module 98, the apposition module 100, and the pore density module 102 from **Figure 13.**

The initialization module 88, the smoothing module 90, the bulge module 92, the rate and limit module 94, the deployment module 96, the push force module 98, the apposition module 100, and/or the pore density module 102 can each comprise, inter alia, a central processing unit (CPU) 110, a memory 112, and a display 114. A bus input/output (I/O) interface 116 couples the initialization module 88, the smoothing module 90, the bulge module 92, the rate and limit module 94, the deployment module 96, the push force module 98, the apposition module 100, and/or the pore density module 102. In certain embodiments, each of the initialization module 88, the smoothing module 90, the bulge module 92, the rate and limit module 94, the deployment module 96, the push force module 98, the apposition module 100, and/or the pore density module 102 are generally coupled to various input devices 118 such as a mouse and keyboard through the bus I/O interface 116 to the display 114. In certain embodiments, one or more of the initialization module 88, the smoothing module 90, the bulge module 92, the rate and limit module 94, the deployment module 96, the push force module 98, the apposition module 100, and/or the pore density module 102 share a common input device 118 and/or display 114. In certain embodiments, the input device 118 is the same as the user input 86 described with respect to Figure 13. In certain embodiments, the display 114 is the same as the display device 84 described with respect to **Figure 13.**

The bus I/O interface 116 can include circuits such as cache, power supplies, clock circuits, and a communications bus. The memory 112 can include RAM, ROM, disk drive, tape drive, etc., or a combination thereof. Exemplary disclosed embodiments may be implemented as a subroutine, routine, program, software, stored in the memory 112 (e.g., a non-transitory computer-readable storage medium) and executed by the CPU 110 to process data. As such, any of the initialization module 88, the smoothing module 90, the bulge module 92, the rate and limit module 94, the deployment module 96, the push force module 98, the apposition module 100, and/or the pore density module 102 can be implemented as a general-purpose computer system that becomes a specific purpose computer system when executing the subroutine, routine, program, software, stored in the memory 112.

In certain embodiments, any of the initialization module 88, the smoothing module 90, the bulge module 92, the rate and limit module 94, the deployment module 96, the push force module 98, the apposition module 100, and/or the pore density module 102 can also include an operating system and micro-instruction code. The various processes and functions described herein may either be part of the micro-instruction code or part of the application program (or a combination thereof) which is executed via the operating system. In addition, various other peripheral devices may be connected to the computer system 80 such as an additional data storage device and a printing device.

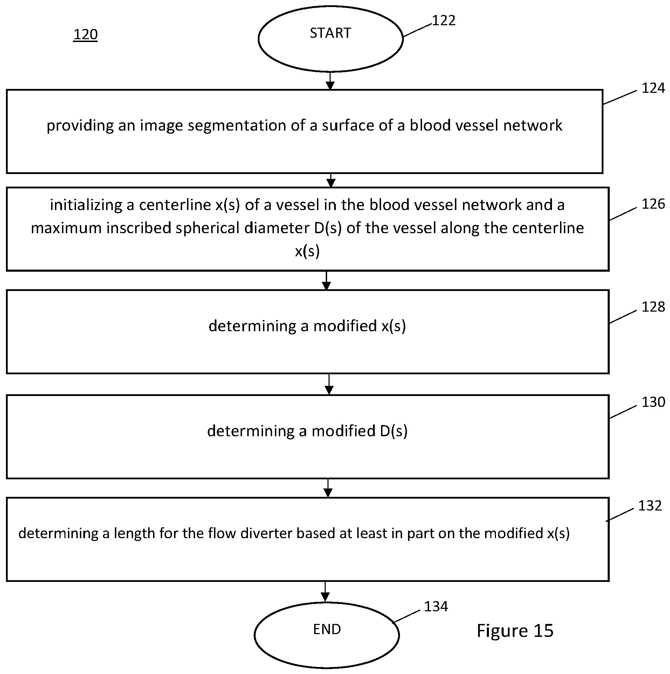

**Figure 15** illustrates a method 120 performed by the system 80 for selecting a correct flow diverter (FD) for a specific patient by predicting deployment of the FD in the blood vessel of the patient in real-time. The method begins at step 122. The method then moves to step 124 where the system 80 receives an image segmentation of a surface of a blood vessel network. The method continues to step 126 where the system 80 initializing a centerline x(s) of a vessel in the blood vessel network and a maximum inscribed spherical diameter D(s) of the vessel along the centerline x(s).

The method continues to step 128 where the system 80 determines a modified x(s). Modifying x(s) can be by smoothing x(s) and D(s) based at least in part on a cubic spline; identifying one or more bulge segments along x(s); and/or replacing each of the one or more bulge segments with a direct path.

The method continues to step 130 where the system 80 determines a modified D(s). In certain embodiments, the modified D(s) is determined based on one or more of a rate and a limit. The method continues to step 132 where the system 80 determines a length for the flow diverter based at least in part on the modified x(s). The method then ends at step 134.

The terms "processor", as used herein is a broad term, and is to be given its ordinary and customary meaning to a person of ordinary skill in the art (and is not to be limited to a special or customized meaning), and refer without limitation to a computer system, state machine, processor, or the like designed to perform arithmetic or logic operations using logic circuitry that responds to and processes the basic instructions that drive a computer. In some embodiments, the terms can include ROM and/or RAM associated therewith.

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The various operations of methods described above may be performed by any suitable means capable of performing the operations, such as various hardware and/or software component(s), circuits, and/or module(s). Generally, any operations illustrated in the Figures may be performed by corresponding functional means capable of performing the operations.

The various illustrative logical steps, blocks, modules and circuits described in connection with the present disclosure may be implemented or performed with a general purpose processor, GPU computational units (using CUDA or OpenCL), or part of the computation may be performed on a digital signal processor (DSP), an application specific integrated circuit (ASIC), a field programmable gate array signal (FPGA) or other programmable logic device (PLD), discrete gate or transistor logic, discrete hardware components or any combination thereof designed to perform the functions described herein. A general purpose processor may be a microprocessor, but in the alternative, the processor may be any commercially available processor, controller, microcontroller or state machine. A processor may also be implemented as a combination of computing devices, e.g., a combination of a DSP and a microprocessor, a plurality of microprocessors, one or more microprocessors in conjunction with a DSP core, or any other such configuration.

In one or more aspects, the functions described may be implemented in hardware, software, firmware, or any combination thereof. If implemented in software, the functions may be stored on or transmitted over as one or more instructions or code on a computer-readable medium. Computer-readable media includes both computer storage media and communication media including any medium that facilitates transfer of a computer program from one place to another. A storage media may be any available media that can be accessed by a computer. By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and blu-ray disc where disks usually reproduce data magnetically, while discs reproduce data optically with lasers. Thus, in some aspects computer readable medium may comprise non-transitory computer readable medium (e.g., tangible media). In addition, in some aspects computer readable medium may comprise transitory computer readable medium (e.g., a signal). Combinations of the above should also be included within the scope of computer-readable media.

The methods disclosed herein comprise one or more steps or actions for achieving the described method. The method steps and/or actions may be interchanged with one another without departing from the scope of the claims. In other words, unless a specific order of steps or actions is specified, the order and/or use of specific steps and/or actions may be modified without departing from the scope of the claims.

Thus, certain aspects may comprise a computer program product for performing the operations presented herein. For example, such a computer program product may comprise a computer readable medium having instructions stored (and/or encoded) thereon, the instructions being executable by one or more processors to perform the operations described herein. For certain aspects, the computer program product may include packaging material.

Software or instructions may also be transmitted over a transmission medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of transmission medium.

Further, it should be appreciated that modules and/or other appropriate means for performing the methods and techniques described herein can be downloaded and/or otherwise obtained by an electronic communication device as applicable. For example, such a device can be coupled to a server to facilitate the transfer of means for performing the methods described herein. Alternatively, various methods described herein can be provided via storage means (e.g., RAM, ROM, a physical storage medium such as a compact disc (CD) or floppy disk, etc.), such that an electronic communication device can obtain the various methods upon coupling or providing the storage means to the device. Moreover, any other suitable technique for providing the methods and techniques described herein to a device can be utilized.

It is to be understood that the claims are not limited to the precise configuration and components illustrated above. Various modifications, changes and variations may be made in the arrangement, operation and details of the methods and apparatus described above without departing from the scope of the claims.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention, e.g., as including any combination of the listed items, including single members (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

Headings are included herein for reference and to aid in locating various sections. These headings are not intended to limit the scope of the concepts described with respect thereto. Such concepts may have applicability throughout the entire specification.

The foregoing illustrated embodiments have been provided solely for illustrating the functional principles of the present invention and are not intended to be limiting. For example, the present invention may be practiced using different overall structural configuration and materials. Persons skilled in the art will appreciate that modifications and alterations of the embodiments described herein can be made without departing from the spirit, principles, or scope of the present invention. The present invention is intended to encompass all modifications, substitutions, alterations, and equivalents within the spirit and scope of the disclosure.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

REPRESENTATIVE FEATURES OF THE DISCLOSURE:

1. A method for real-time sizing of flow diverters for cerebral aneurysm treatment, the method comprising:

providing an image segmentation of a surface of a blood vessel network,
initializing a centerline x(s) of a vessel in the blood vessel network and a maximum inscribed spherical diameter D(s) of the vessel along the centerline x(s);
determining a modified x(s) by,

smoothing x(s) and D(s) based at least in part on a cubic spline, identifying one or more bulge segments along x(s), and
replacing each of the one or more bulge segments with a direct path segment; and

determining a modified D(s) based on one or more of a rate and a limit.

2. The method of Clause 1, further comprising determining a length for the flow diverter based at least in part on the modified x(s).

3. The method of Clause 1, further comprising determining a length for the flow diverter based at least in part on the equation

$$L_0 = \int_0^{3} \frac{\sin(\beta_0)}{\sqrt{1 - (D(s)\cos(\beta_0)/D_0)^2}} ds$$

.

4. The method of Clause 1, further comprising determining a change in the length based at least in part on a push force.

5. The method of Clause 1, further comprising determining a ratio of a cross-sectional area of the flow diverter to a cross-sectional area of the vessel at each point along the modified x(s).

6. The method of Clause 1, wherein the direct path segment is a more direct smooth path which is still unaffected by the vessel.

7. The method of Clause 1, wherein the direct path segment is a more likely path that will be followed by the flow diverter.

8. The method of Clause 1, further comprising determining a pore density of a surface of the flow diverter.

9. A system for real-time sizing of flow diverters for cerebral aneurysm treatment, the system comprising:
one or more processors configured to:

provide an image segmentation of a surface of a blood vessel network,
initialize a centerline x(s) of a vessel in the blood vessel network and a maximum inscribed spherical diameter D(s) of the vessel along the centerline x(s);
determine a modified x(s) by,

smoothing x(s) and D(s) based at least in part on a cubic spline,
identifying one or more bulge segments along x(s), and
replacing each of the one or more bulge segments with a direct path segment; and

determine a modified D(s) based on one or more of a rate and a limit.

10. The system of Clause 9, wherein the one or more processors is further configured to determine a length for the flow diverter based at least in part on the modified x(s).

11. The system of Clause 9, wherein the one or more processors is further configured to determine a length for the flow diverter based at least in part on the

$$L_0 = \int_0^S \frac{\sin(\beta_0)}{\sqrt{1 - (D(s)\cos(\beta_0)/D_0)^2}} ds$$

equation

12. The system of Clause 9, wherein the one or more processors is further configured to determine a change in the length based at least in part on a push force.

13. The system of Clause 9, wherein the one or more processors is further configured to determine a ratio of a cross-sectional area of the flow diverter to a cross-sectional area of the vessel at each point along the modified x(s).

14. The system of Clause 9, wherein the direct path segment is a more direct smooth path which is still unaffected by the vessel.

15. The system of Clause 9, wherein the direct path segment is a more likely path that will be followed by the flow diverter.

16. The system of Clause 9, wherein the one or more processors is further configured to determine a pore density of a surface of the flow diverter.

17. A non-transitory computer readable storage medium having stored thereon instructions that, when executed, cause a computing device to:

receive an image segmentation of a surface of a blood vessel network,
initialize a centerline x(s) of a vessel in the blood vessel network and a maximum inscribed spherical diameter D(s) of the vessel along the centerline x(s);
determine a modified x(s) by,

smoothing x(s) and D(s) based at least in part on a cubic spline,
identifying one or more bulge segments along x(s), and
replacing each of the one or more bulge segments with a direct path segment; and

determine a modified D(s) based on one or more of a rate and a limit.

18. The non-transitory computer readable storage medium of Clause 17, wherein the instructions, when executed, cause the at least one computing device to determine a length for the flow diverter based at least in part on the modified x(s).

19. The non-transitory computer readable storage medium of Clause 17, wherein the instructions, when executed, cause the at least one computing device to determine a length for the flow diverter based at least in part on the equation

$$L_0 = \int_0^S \frac{\sin(\beta_0)}{\sqrt{1 - (D(s)\cos(\beta_0)/D_0)^2}} ds$$

20. The non-transitory computer readable storage medium of Clause 17, wherein the instructions, when executed, cause the at least one computing device to determine a change in the length based at least in part on a push force.

21. A method for real-time sizing of flow diverters for cerebral aneurysm treatment, the method comprising:

providing an image segmentation of a surface of a blood vessel network;

initializing a centerline x(s) of a vessel in the blood vessel network and a maximum inscribed predefined geometric shape D(s) of the vessel along the centerline x(s);
determining a modified x(s) by,

smoothing x(s) and D(s),
identifying one or more bulge segments along x(s), and
replacing each of the one or more bulge segments with a direct path segment; and

determining a modified D(s).

22. A system for real-time sizing of flow diverters for cerebral aneurysm treatment, the system comprising:
one or more processors configured to:

provide an image segmentation of a surface of a blood vessel network,
initializing a centerline x(s) of a vessel in the blood vessel network and a maximum inscribed predefined geometric shape D(s) of the vessel along the centerline x(s);
determining a modified x(s) by,

smoothing x(s) and D(s),
identifying one or more bulge segments along x(s), and
replacing each of the one or more bulge segments with a direct path segment; and

determining a modified D(s).

**Claims**

1. A computer-implemented method for real-time sizing of flow diverters for cerebral aneurysm treatment, the method comprising:

providing an image segmentation of a surface of a blood vessel network;
initializing a centerline x(s) of a vessel in the blood vessel network and a maximum inscribed predefined geometric shape D(s) of the vessel along the centerline x(s);
determining a modified x(s) by,

smoothing x(s) and D(s),
identifying one or more bulge segments along x(s), and
replacing each of the one or more bulge segments with a direct path segment; and

determining a modified D(s).

2. The method of Claim 1, wherein the predefined geometric shape is a spherical diameter and the method further comprises:

smoothing x(s) and D(s) is based at least in part on a cubic spline; and
determining the modified D(s) is based on one or more of a rate and a limit.

3. The method of Claim 2, further comprising determining a length for the flow diverter based at least in part on the modified x(s).

4. The method of Claim 2 or Claim 3, further comprising determining a length for the flow diverter based at least in part on the equation

$$L_0 = \int_0^S \frac{\sin(\beta_0)}{\sqrt{1-(D(s)cos(\beta_0)/D_0)^2}} ds.$$

5. The method of any one of Claims 2 to 4, further comprising determining a change in the length based at least in part on a push force.

6. The method of any one of Claims 2 to 5, further comprising determining a ratio of a cross-sectional area of the flow diverter to a cross-sectional area of the vessel at each point along the modified x(s).

7. The method of any one of Claims 2 to 6, wherein the direct path segment is a more direct smooth path which is still unaffected by the vessel.

8. The method of any one of claims 2 to 6, wherein the direct path segment is a more likely path that will be followed by the flow diverter.

9. The method of any one of Claims 2 to 8, further comprising determining a pore density of a surface of the flow diverter.

10. A system for real-time sizing of flow diverters for cerebral aneurysm treatment, the system comprising one or more processors and a memory, wherein the system is configured to perform the method of any one of claims 1 to 9.

11. A computer program having instructions which, when executed by a computing system, cause the computing system to perform the method of any one of claims 1 to 9.

12. A computer readable medium storing a computer program according to claim 11.

Figure 1

Figure 2

Figure 3

Figure 4

Figure 5

Figure 6

Figure 7

Figure 8

Figure 9

Figure 10

Figure 11

Figure 12

80

86 — User Input

84 — Display Device

82 — Image Apparatus

88 — Initialization Module

90 — Smoothing Module

92 — Bulge Module

94 — Rate and Limit Module

96 — Deployment Module

98 — Push Force Module

100 — Apposition Module

102 — Pore Density Module

Figure 13

88, 90, 92, 94, 96,
98, 100, 102

| | | |
|---|---|---|
| Input | | CPU |

118

110

Display Device

114

116

MEMORY

112

Figure 14

120

START — 122

providing an image segmentation of a surface of a blood vessel network — 124

initializing a centerline x(s) of a vessel in the blood vessel network and a maximum inscribed spherical diameter D(s) of the vessel along the centerline x(s) — 126

determining a modified x(s) — 128

determining a modified D(s) — 130

determining a length for the flow diverter based at least in part on the modified x(s) — 132

END — 134

Figure 15

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2012/323547 A1 (BALOCH SAJJAD HUSSAIN [US] ET AL) 20 December 2012 (2012-12-20)<br>* abstract *<br>* figures 3,5,6,9 *<br>* paragraphs [0030] - [0034], [0038] *<br>----- | 1-3,5-12<br>4 | INV.<br>G06T7/00<br>G06T7/62 |
| A | EP 2 749 222 A1 (EBM CORP [JP])<br>2 July 2014 (2014-07-02)<br>* abstract *<br>* paragraph [0019] *<br>----- | 9 | |
| A,D | JEDWAB M R ET AL: "A STUDY OF THE GEOMETRICAL AND MECHANICAL PROPERTIES OF A SELF-EXPANDING METALLIC STENT-THEORY AND EXPERIMENT",<br>JOURNAL OF APPLIED BIOMATERIALS, JOHN WILEY & SONS, INC., NEW YORK, NY, US,<br>vol. 4, 1 January 1993 (1993-01-01), pages 77-85, XP009027866,<br>ISSN: 1045-4861, DOI: 10.1002/JAB.770040111<br>* abstract *<br>* equations 4-9 *<br>----- | 4 | |
| A | EP 3 025 638 A1 (GALGO MEDICAL S L [ES])<br>1 June 2016 (2016-06-01)<br>* abstract *<br>* paragraph [0031] *<br>----- | 4 | |

TECHNICAL FIELDS
SEARCHED      (IPC)

G06T
G06K

The present search report has been drawn up for all claims

| Place of search | Date of completion of the search | Examiner |
|---|---|---|
| Munich | 11 November 2020 | Scholz, Volker |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

# EP 3 770 852 A1

## ANNEX TO THE EUROPEAN SEARCH REPORT
## ON EUROPEAN PATENT APPLICATION NO.

EP 20 18 7432

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2020

| Patent document cited in search report | | | Publication date | Patent family member(s) | | | Publication date |
|---|---|---|---|---|---|---|---|
| US 2012323547 | A1 | | 20-12-2012 | NONE | | | |
| EP 2749222 | A1 | | 02-07-2014 | CA | 2850189 | A1 | 07-03-2013 |
| | | | | CA | 2850457 | A1 | 07-03-2013 |
| | | | | CA | 2850476 | A1 | 07-03-2013 |
| | | | | CA | 2850691 | A1 | 07-03-2013 |
| | | | | CN | 103917164 | A | 09-07-2014 |
| | | | | CN | 103917165 | A | 09-07-2014 |
| | | | | CN | 103930036 | A | 16-07-2014 |
| | | | | CN | 103930037 | A | 16-07-2014 |
| | | | | EP | 2749222 | A1 | 02-07-2014 |
| | | | | EP | 2749223 | A1 | 02-07-2014 |
| | | | | EP | 2749224 | A1 | 02-07-2014 |
| | | | | EP | 2749225 | A1 | 02-07-2014 |
| | | | | JP | 5596865 | B2 | 24-09-2014 |
| | | | | JP | 5596866 | B2 | 24-09-2014 |
| | | | | JP | 5596867 | B2 | 24-09-2014 |
| | | | | JP | 6097912 | B2 | 22-03-2017 |
| | | | | JP | 6535859 | B2 | 03-07-2019 |
| | | | | JP | 2017113578 | A | 29-06-2017 |
| | | | | JP | WO2013031741 | A1 | 23-03-2015 |
| | | | | JP | WO2013031742 | A1 | 23-03-2015 |
| | | | | JP | WO2013031743 | A1 | 23-03-2015 |
| | | | | JP | WO2013031744 | A1 | 23-03-2015 |
| | | | | KR | 20140074904 | A | 18-06-2014 |
| | | | | KR | 20140082663 | A | 02-07-2014 |
| | | | | KR | 20140082664 | A | 02-07-2014 |
| | | | | KR | 20150000450 | A | 02-01-2015 |
| | | | | US | 2014316758 | A1 | 23-10-2014 |
| | | | | US | 2014343906 | A1 | 20-11-2014 |
| | | | | US | 2015032435 | A1 | 29-01-2015 |
| | | | | US | 2015127031 | A1 | 07-05-2015 |
| | | | | WO | 2013031741 | A1 | 07-03-2013 |
| | | | | WO | 2013031742 | A1 | 07-03-2013 |
| | | | | WO | 2013031743 | A1 | 07-03-2013 |
| | | | | WO | 2013031744 | A1 | 07-03-2013 |
| EP 3025638 | A1 | | 01-06-2016 | EP | 3025638 | A1 | 01-06-2016 |
| | | | | ES | 2459244 | A1 | 08-05-2014 |
| | | | | US | 2016232659 | A1 | 11-08-2016 |
| | | | | WO | 2015063352 | A1 | 07-05-2015 |

EPO FORM P0459

For more details about this annex : see Official Journal of the European Patent Office, No. 12/82

34

EP 3 770 852 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 16516136 B
- US 16516150 B
- US 16516140 B

**Non-patent literature cited in the description**

- **BRIGANTI F ; DELEHAYE L ; LEONE G et al.** Flow diverter device for the treatment of small middle cerebral artery aneurysms. *J Neurointerv Surg.,* 2016, vol. 8 (3), 287-294
- **BHOGAL P ; PEREZ MA ; GANSLANDT O ; BÄZNER H ; HENKES H ; FISCHER S.** Treatment of posterior circulation non-saccular aneurysms with flow diverters: a single-center experience and review of 56 patients. *J Neurointerv Surg.,* 2017, vol. 9 (5), 471-481
- **SAATCI I ; YAVUZ K ; OZER C ; GEYIK S ; CEKIRGE HS.** Treatment of intracranial aneurysms using the pipeline flow-diverter embolization device: a single-center experience with long-term follow-up results. *American Journal of Neuroradiology,* 2012, vol. 33 (8), 1436-1446
- P100018/S015 Pipeline™ Flex Embolization Device Approval Letter. *U.S. Food and Drug Administration,* 25 January 2019, https://www.accessdata.fda.gov/cdrh_docs/pdf10/P100018S015A.pdf.
- **CAROFF J ; TAMURA T ; KING RM et al.** Phosphorylcholine surface modified flow diverter associated with reduced intimal hyperplasia. *J Neurointerv Surg.,* 2018, vol. 10 (11), 1097-1101
- **LAURENT G ; MAKOYEVA A ; DARSAUT TE et al.** In vitro reproduction of device deformation leading to thrombotic complications and failure of flow diversion. *Interv Neuroradiol.,* vol. 19 (4), 432-437
- **TSAI Y-H ; WONG H-F ; HSU S-W.** Endovascular management of spontaneous delayed migration of the flow-diverter stent. *J Neuroradiol.,* December 2018
- **AL-MUFTI F ; AMULURU K ; COHEN ER et al.** Rescue therapy for procedural complications associated with deployment of flow-diverting devices in cerebral aneurysms. *Oper Neurosurg.,* 2018, vol. 15 (6), 624-633
- **NARATA AP ; BLASCO J ; ROMAN LS et al.** Early results in flow diverter sizing by computational simulation: quantification of size change and simulation error assessment. *Oper Neurosurg.,* 2018, vol. 15 (5), 557-566
- **JEDWAB MR ; CLERC CO.** A study of the geometrical and mechanical properties of a self-expanding metallic stent-theory and experiment. *Journal of Applied Biomaterials,* 1993, vol. 4 (1), 77-85